# EUROPEAN PATENT APPLICATION

(11) **EP 3 178 392 A1**
(43) Date of publication of application: **14.06.2017**
(21) Application number: 15306961.2
(22) Date of filing: 09.12.2015
(51) Int. Cl.: A61B 5/0478, A61B 5/00, A61B 5/0484, A61B 1/00

(54) **AUTONOMOUS BIOELECTRIC PHYSIOLOGICAL SIGNAL ACQUISITION DEVICE**

(71) Applicant: RYTHM, 75009 Paris (FR)
(72) Inventor: MOHAMADABADI, Kaveh, 75016 Paris (FR); MERCIER, Hugo, 91380 Chilly Mazarin (FR); SOULET DE BRUGIERE, Quentin, 33115 Pyla sur Mer (FR)
(74) Representative: Cabinet Plasseraud

(57) **Abstract**

An autonomous bioelectric physiological signal acquisition device comprises an electrode (3) acquiring an analog measurement signal, analog signal processing and analog-digital converter chips (9) filtering and converting the analog measurement signal, a wireless communication module (12), a microprocessor (13) controlling the wireless communication module and a battery (15).

The device further comprises a microcontroller (10) and a temporary memory (11), and a first printed circuit board (5) and a second printed circuit board (6) thermally separated from each other.

The device is such that the electrode (3), the analog signal processing and analog-digital converter chips (9), the microcontroller (10) and the temporary memory (11) are disposed on the first printed circuit board (5), and the wireless communication module (12) and the microprocessor (13) are disposed on the second printed circuit board (6).

## Description

### FIELD OF THE INVENTION

The instant invention relates to autonomous bioelectric physiological signal acquisition devices and methods for acquiring a bioelectric physiological signal using such autonomous bioelectric physiological signal acquisition devices.

### BACKGROUND OF THE INVENTION

Bioelectric physiological signals generated by electrical currents within the neurons or the nerves of an individual can be detected by performing electrocardiogram or electroencephalogram for instance. Such measurements have led to breakthrough in the field of biological sciences and present huge potentials in many daily-life fields such as self-quantization, self-optimization or human-computer interfaces to name only a few.

Recently, bioelectric physiological signal acquisition devices have started to be developed with the aim of providing conformable and easy to use way of acquiring bioelectric physiological signal.

However, the quality and the reliability of the acquisition is directly dependant on the performance of such devices.

Indeed, bioelectric physiological signals are very low level signals, in the millivolt to microvolt range, and their acquisition in a daily life environment and with a small foot-print autonomous device is difficult to perform with a low noise.

Documents WO 2015/017563, CN 103961093, CN 102512159 and WO 2014062738 describe examples of such devices.

These devices usually comprise a few electrodes to acquire analog signals, amplifiers and converters to obtain a digital signal and a microprocessor controlling a wireless communication module to receive and transmit the signals.

While these solutions are quite miniaturized, there is a need to further improve the signal over noise ratio of the acquisition as well as the quality and the reliability of the bioelectric physiological signals acquisition.

The instant invention has notably for object to improve this situation.

### SUMMARY OF THE INVENTION

To this aim, a first object of the invention is an autonomous bioelectric physiological signal acquisition device comprising
at least one electrode acquiring an analog measurement signal representative of a bioelectric physiological signal,
analog signal processing and analog-digital converter chips filtering and converting the analog measurement signal in a digital measurement signal,
a wireless communication module and a microprocessor controlling the wireless communication module,
at least one battery powering the analog signal processing and analog-digital converter chips, the wireless communication module and the microprocessor.

The device further comprises a microcontroller and a temporary memory storing the digital measurement signal and communicating the digital measurement signal to the microprocessor, and a first printed circuit board and a second printed circuit board thermally separated from each other.

The device is such that at least one electrode, the analog signal processing and analog-digital converter chips, the microcontroller and the temporary memory are disposed on the first printed circuit board, and the wireless communication module and the microprocessor are disposed on the second printed circuit board.

In some embodiments, one might also use one or more of the following features:
- the device further comprises a third printed circuit board separated from the first printed circuit board and from the second printed circuit board, and
   the first, the second and the third printed circuit boards are connected in series, the third printed circuit board being disposed between the first printed circuit board and the second printed circuit board;
- the third printed circuit board comprises a USB connector and at least one battery charge chip able to receive energy from the USB connector and charges said at least one battery;
- the device further comprises at least one power path chip and at least two batteries connected to the power path chip, preferably wherein the at least one power path chip is disposed on the third printed circuit board;
- the device further comprises a power management chip disposed on the second printed circuit board and able to power at the wireless communication module and the microprocessor, preferably wherein the power management chip is connected to the power path chip;
- the device comprises a first power step-up chip to power the analog signal processing and analog-digital converter chips, preferably wherein said first power step-up chip is located on the third printed circuit board or is located on the first printed circuit board and is isolated from a ground plane of the analog signal processing and analog-digital converter chips;
- the printed circuit boards are connected by low thermal conduction flat flexible elements, in particular ribbon cables or Flat Flexible Cables;
- the wireless communication module comprises a RF transceiver chip able to transmit and receive data using the Wi-Fi and the Bluetooth standards;
- the analog signal processing and analog-digital converter chips form a single monolithic chip disposed on the first printed circuit board;
- the analog-digital converter has a 24bit delta-sigma (Δ-Σ) type ADC, an output data rate between 250SPS and 32kSPS and at least 4 channels;
- the device comprises 4 electrodes;
- the device comprises at least one acoustic transducer able to generate an acoustic stimulation and at least one codec module able to convert a digital stimulation signal in an analog stimulation signal to drive the acoustic transducer, preferably wherein said codec module is disposed on the first printed circuit card;
- the device comprises a second power step-up chip to power the codec module, preferably wherein said second power step-up chip is located on the third printed circuit board or is located on the second printed circuit board.

Another object of the invention is a method for acquiring a bioelectric physiological signal using an autonomous bioelectric physiological signal acquisition device as detailed above, wherein:
- an autonomous bioelectric physiological signal acquisition device as detailed above is provided and worn by an individual,
- the at least one electrode is located on the individual in order to continuously acquire an analog measurement signal representative of a bioelectric physiological signal,
- the analog signal processing and analog-digital converter chips continuously filter and convert the analog measurement signal in a digital measurement signal,
- the microcontroller continuously receive a digital measurement signal from the analog signal processing and analog-digital converter chips and store said signal in a temporary memory, and
- the microprocessor is periodically awakened from a sleep state to process the signal stored in the temporary memory.

According to an embodiment of this method, if the microprocessor detects that the signal recorded in the temporary memory is representative of a stimulation-ready state of the individual, the microprocessor is continuously kept in an awaken state and command the acoustic transducer and the codec module to generate acoustic stimulations to the individual.

With these features, among other advantages, the signal over noise ratio of the acquisition as well as the quality and the reliability of the bioelectric physiological signals acquisition can be increased.

### BRIEF DESCRIPTION OF THE DRAWINGS

Other characteristics and advantages of the invention will readily appear from the following description of several of its embodiments, provided as non-limitative examples, and of the accompanying drawings.

On the drawings:
- Fig. 1 is a schematic view in perspective of an autonomous bioelectric physiological signal acquisition device according to an embodiment of the invention,
- Fig. 2 is a detailed schematic view of the device of figure 1 seen from the top, showing the control module and the batteries of the device,
- Fig. 3 is a detailed view of the control module of the device of figure 1, showing a schematic of printed circuit boards and their main components.

On the different figures, the same reference signs designate like or similar elements.

### DETAILED DESCRIPTION

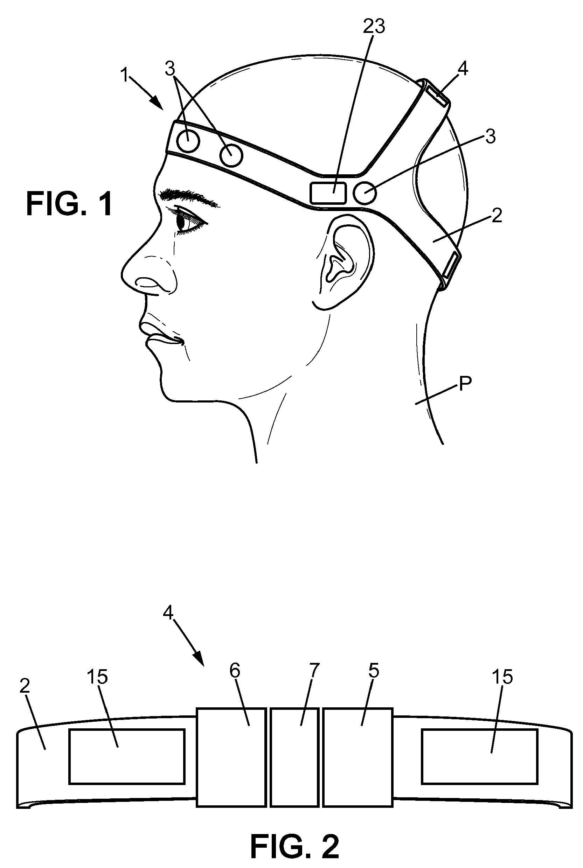

Figure 1 illustrates an autonomous bioelectric physiological signal acquisition device 1 according to an embodiment of the invention.

The device 1 is adapted to be worn by an individual, for example during a sleep period of the individual.

The device 1 is for example adapted to be worn on the head of the individual.

To this end, the device 1 can comprise one or more support members 2 adapted to at least partially surround the head of the individual so as to be held therein. In the embodiments of figure 1, the supports members 2 comprise three branches arranged so as to surround the head of the individual and maintain the device 1.

Alternatively, the device 1 may also be divided into elements, adapted to be worn on various parts of the body of the individual, for example on the head or wrist or on the chest.

This device 1 further comprises four electrodes 3 able to be in contact with the skin of the individual and to acquire an analog measurement signal. The analog measurement signal is advantageously representative of a bioelectric physiological signal.

Electrodes 3 may be reusable electrodes or disposable electrodes. Advantageously, electrodes 3 are reusable electrodes so as to simplify the daily use of the device. Electrodes 3 may be, in particular, dry electrodes or electrodes coated with a contact gel. The electrodes 2 may also be of textile or silicone electrodes.

In one embodiment of the invention, the device 1 comprises five electrodes 3 arranged, for example on the surface of the scalp of the person P.

The five electrodes 3 may comprise for instance two reference electrodes located close to the mastoids, two measurement electrodes located on the forehead and a bias or virtual ground electrode that may also be located on the forehead. Such an arrangement is only described as a manner of example and should not be understood as being limitative with respect to the invention.

The five electrodes 3 are held by the supports members 2.

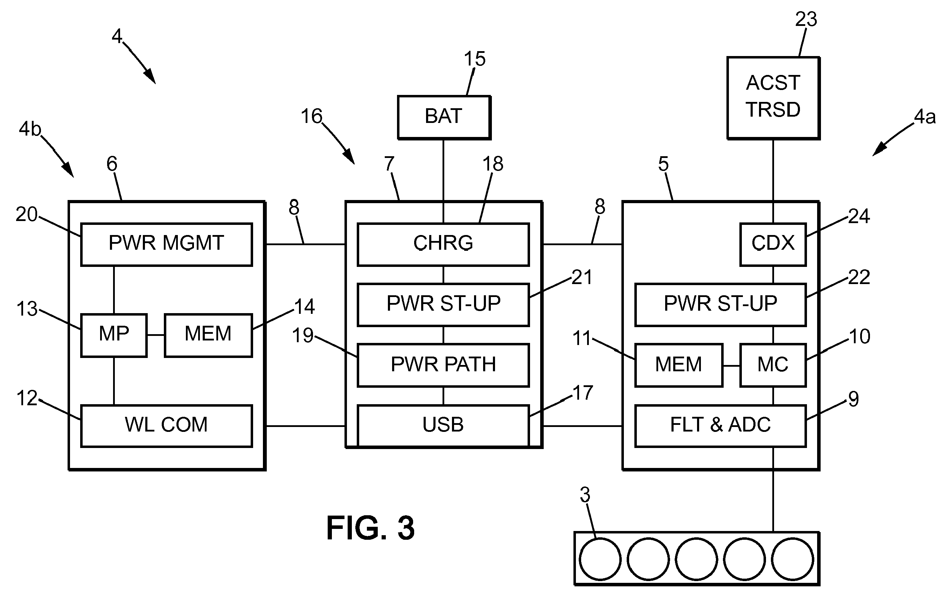

As illustrated on figures 2 and 3, the device 1 further comprises a control system 4 to process and store the signals of the electrodes.

The control system 4 comprise three printed circuit boards 5, 6, 7, a first printed circuit board 5, a second first printed circuit board 6 and a third first printed circuit board 7.

The three printed circuit boards 5, 6, 7 are also held by the supports members 2.

As illustrated on figures 2 and 3, the first, the second and the third printed circuit boards 5, 6, 7 are connected in series. The third printed circuit board 7 is disposed between the first printed circuit board 5 and the second printed circuit board 6.

The three printed circuit boards 5, 6, 7 are separated from one another.

The printed circuit boards 5, 6, 7 are connected by low thermal conduction flat flexible elements 8. The flat flexible elements 8 are made of plastic and can incorporate conductive electric tracks.

In one example, the flat flexible elements 8 can be ribbon cables or Flat Flexible Cables or Flexible Printed Circuit.

This way the three printed circuit boards 5, 6, 7 are thermally isolated from one another.

Each printed circuit boards 5, 6, 7 has a particular function, a particular way of operating and the electronic components of the control system 4 are thus arranged on the three board as it will now be detailed.

The first printed circuit board 5 comprises the acquisition and stimulation module 4a of the control system 4.

More precisely, the control system 4 comprises analog signal processing and analog-digital converter chips 9 connected to the electrodes 3.

The analog signal processing and analog-digital converter chips 9 are disposed on the first printed circuit board 5 and receive the analog measurement signals from the electrodes 3.

The analog signal processing and analog-digital converter chips 9 comprise analog signal processing modules and analog-digital converter modules.

The analog signal processing and analog-digital converter chips 9 may form a single monolithic chip 9 disposed on the first printed circuit board 5. One example of thus a chip is an analog front-end module with reference ADS1294 of Texas Instrument®.

The analog signal processing module comprises filters able to filter the analog measurement signal, for instance low-pass and/or high-pass filters.

The analog signal processing module also comprises analog signal amplifiers.

The input referred noise of the amplifiers may be around 4uV at 150 Hz bandwidth. The amplifiers may thus have a high input impedance, high common mode rejection ratio, low bias current and low noise to reduce the mentioned noise sources. The amplifiers may be programmable gain amplifiers with an adjustable gain, for instance adjustable to at least a 12 times amplification.

The analog-digital converter modules are able to convert the filtered analog measurement signal in a digital measurement signal. The analog-digital converters may have at least 4 channels and comprises 24bit delta-sigma (Δ-Σ) type ADC with an output data rate between 250SPS and 32kSPS.

Each analog signal processing module of the analog signal processing and analog-digital converter chips 9 may advantageously receive signals from two electrodes 3, among the plurality of electrodes 5, in order to perform a differential signal measurement.

The acquisition and stimulation module 4a further comprises a microcontroller 10 and a temporary memory 11.

The microcontroller 10 is able to continuously receive the digital measurement signal from the analog signal processing and analog-digital converter chips 9.

The microcontroller 10 is able to continuously store the digital measurement signal in the temporary memory 11.

The microcontroller 10 may be a low power microcontroller. One example of low power microcontroller is a Cortex®-M0+ of ARM®.

The second printed circuit board 6 comprises the processing and communication module 4b of the control system 4.

More precisely, the device 1 comprises a wireless communication module 12 and a microprocessor 13.

The wireless communication module 12 and a microprocessor 13 are disposed on the second printed circuit board 6.

The microprocessor 13 is able to communicate with the microcontroller 10 in order to receive a digital measurement signal and to control the wireless communication module 12. Said digital measurement signal may be stored in the temporary memory 11 or may be transmitted directly by the microcontroller 10 as it is received from the analog signal processing and analog-digital converter chips 9.

The microprocessor 13 may be able to achieve 2.5DMIPS/MHZ (with frequency at 1 GHz). The microprocessor 13 may be for instance a cortex A9 Series Processor of ARM®.

In one embodiment of the invention, the microprocessor 13 is thus periodically awakened from a sleep state to process the signal stored in the temporary memory 11.

The microprocessor 13 can then store the signal in a second memory 14, disposed on the second printed circuit board 6. The second memory 14 may have a larger size than the temporary memory 11. The second memory 14 may comprise a double data rate (DDR) memory and an embedded multi-media controller (eMMC).

The wireless communication module 12 may comprise a RF transceiver chip 12 able to transmit and receive data using the Wi-Fi and the Bluetooth standards.

Since the processing and module 4b has significantly more power consumption compared to the acquisition and stimulation module 4a, the microprocessor may be used in case of need or periodically.

The temperature on the second printed circuit board 6 can thus vary over time.

Since the first printed circuit board 5 and the second printed circuit board 6 are separated, the effect of these thermal variations is minimal on the acquisition and stimulation module 4a.

Moreover, the device 1 comprises at least one battery 15. The battery 15 powers the elements of the device 1, in particular at least the analog signal processing and analog-digital converter chips 9, the wireless communication module 12 and the microprocessor 13.

The device comprises a power module 16 disposed on the second printed circuit board 6 and on the third printed circuit board 7.

On the third printed circuit board 7, the power module 16 may comprise a USB connector 17 and at least one battery charge chip 18.

On the second printed circuit board 6, the power module 16 may comprise a power management chip 20.

The power management chip 20 is able to power the microprocessor 13, the second memory 14 and/or the wireless communication module 12.

The battery charge chip 18 is able to receive energy from the USB connector 17 and charges said at least one battery 15.

In one embodiment, the device 1 may comprise two batteries 15 or more. In this embodiment, the device 1 may then comprise two battery charge chips 18 respectively connected to each battery 15 and may comprise a power-path manager chip 19.

The two batteries 15 may then be both connected to the power-path manager chip 19 of the third printed circuit board 7, through the respective battery charge chips 18.

The power management chip 20 may then receive energy from the power-path manager chip 19.

Moreover, the device can also comprise a first power step-up chip 21.

The first power step-up chip 21 may be able to power the analog signal processing module of the analog signal processing and analog-digital converter chips 9..

The first power step-up chip may be located on the third printed circuit board 7 or on the first printed circuit board 5.

If the first power step-up chip 21 is located on the first printed circuit board 5, it may then be isolated from a ground plane of the analog signal processing and analog-digital converter chips 9.

The first power step-up chip 21 may in particular be a low noise step up chip.

The first power step-up chip 21 may be powered by the power module 16 of the third printed circuit board 7, in particular by the power-path manager chip 19 of the power module 16.

This way the first stage of the acquisition and stimulation module 4a and the processing and communication module 4b are powered separately and isolated from the acquisition part of the device 1 which prevent interferences, noises and thermal influence on the acquisition and stimulation module 4a.

The device 1 may further comprise at least one acoustic transducer 23 able to generate an acoustic stimulation.

In particular, as mentioned here above, microprocessor 13 may be periodically awakened from a sleep state to process the signal stored in the temporary memory 11. If the microprocessor 13 detects that the signal recorded in the temporary memory 11 is representative of a stimulation-ready state of the individual, the microprocessor 13 may then be continuously kept in an awaken state and command the acoustic transducer 23 to generate acoustic stimulations to the individual.

To this aim, the device may further comprise at least one codec module 24 able to convert a digital stimulation signal in an analog stimulation signal to drive the acoustic transducer 23.

The codec module 24 may be disposed on the first printed circuit card 5.

The device can thus also comprise a second power step-up chip 22 to power the codec module 24.

The second power step-up chip 22 may be located on the third printed circuit board 7 or on the first printed circuit board 5 and be isolated from a ground plane of the analog signal processing and analog-digital converter chips 9.

In an alternate embodiment, the second power step-up chip 22 may be located on the second printed circuit board 6.

The second power step-up chip 22 may be powered by the power module 16 of the third printed circuit board 7, in particular by the power-path manager chip 19 of the power module 16.

By virtue of this arrangement of the device, all the acquisition components and electrode connections are fully separated. Moreover, the components that can cause thermal variations are also kept away from the acquisition part. The present device thus allow for a high quality and reliability of the bioelectric physiological signal acquisition and acoustic stimulation of the individual.

## Claims

1. An autonomous bioelectric physiological signal acquisition device comprising
at least one electrode (3) acquiring an analog measurement signal representative of a bioelectric physiological signal,
analog signal processing and analog-digital converter chips (9) filtering and converting the analog measurement signal in a digital measurement signal,
a wireless communication module (12) and a microprocessor (13) controlling the wireless communication module,
at least one battery (15) powering the analog signal processing and analog-digital converter chips (9), the wireless communication module (12) and the microprocessor (13),
**characterized in that** the device further comprises
- a microcontroller (10) and a temporary memory (11) storing the digital measurement signal and communicating the digital measurement signal to the microprocessor (13), and
- a first printed circuit board (5) and a second printed circuit board (6) thermally separated from each other,
the device being further **characterized in that**
- the at least one electrode (3), the analog signal processing and analog-digital converter chips (9), the microcontroller (10) and the temporary memory (11) are disposed on the first printed circuit board (5), and
- the wireless communication module (12) and the microprocessor (13) are disposed on the second printed circuit board (6).

2. Device according to claim 1, **characterized in that** the device further comprises a third printed circuit board (7) separated from the first printed circuit board (5) and from the second printed circuit board (6),
and **in that** the first, the second and the third printed circuit boards (5, 6, 7) are connected in series, the third printed circuit board (7) being disposed between the first printed circuit board (5) and the second printed circuit board (6).

3. Device according to claim 2, wherein the third printed circuit board (7) comprises a connector (17) and at least one battery charge chip (18) able to receive energy from the connector (17) and charges said at least one battery (15), in particular wherein said connector (17) is a USB connector.

4. Device according to claim 2 or 3, wherein the device further comprises at least one power-path manager chip (19) and at least two batteries (15) connected to the power-path manager chip (19), preferably wherein the at least one power-path manager chip (19) is disposed on the third printed circuit board (7).

5. Device according to anyone of claims 1 to 4, wherein the device further comprises a power management chip (20) disposed on the second printed circuit board (6) and able to power the wireless communication module (12) and the microprocessor (13), preferably wherein the power management chip (20) is connected to the power-path manager chip (19).

6. Device according to anyone of claims 1 to 5, comprising a first power step-up chip (21) to power the analog signal processing and analog-digital converter chips (9), preferably wherein said first power step-up chip (21) is located on the third printed circuit board (7) or is located on the first printed circuit board (5) and is isolated from a ground plane of the analog signal processing and analog-digital converter chips (9).

7. Device according to anyone of claims 1 to 6, wherein the printed circuit boards (5, 6, 7) are connected by low thermal conduction flat flexible elements (8), in particular ribbon cables or Flat Flexible Cables.

8. Device according to anyone of claims 1 to 7, wherein the wireless communication module (12) comprises a RF transceiver chip able to transmit and receive data using the Wi-Fi and the Bluetooth standards.

9. Device according to anyone of claims 1 to 8, wherein the analog signal processing and analog-digital converter chips (9) form a single monolithic chip disposed on the first printed circuit board (5).

10. device according to anyone of claims 1 to 9, wherein the analog signal processing and analog-digital converter chips (9) comprise a 24bit delta-sigma (Δ-Σ) type ADC, an output data rate between 250SPS and 32kSPS and at least 4 channels.

11. Device according to anyone of claims 1 to 10, the device comprising 4 electrodes (3).

12. Device according to anyone of claims 1 to 11, further comprising at least one acoustic transducer (23) able to generate an acoustic stimulation and at least one codec module (24) able to convert a digital stimulation signal in an analog stimulation signal to drive the acoustic transducer, preferably wherein said codec module (24) is disposed on the first printed circuit card (5).

13. Device according to claim 12, comprising a second power step-up chip (22) to power the codec module (24), preferably wherein said second power step-up chip (22) is located on the third printed circuit board (7) or is located on the second printed circuit board (6).

14. Method for acquiring a bioelectric physiological signal using an autonomous bioelectric physiological signal acquisition device (1) according to anyone of claims 1 to 13, wherein:
- an autonomous bioelectric physiological signal acquisition device (1) according to anyone of claims 1 to 13 is provided and worn by an individual,
- the at least one electrode (3) is located on the individual in order to continuously acquire an analog measurement signal representative of a bioelectric physiological signal,
- the analog signal processing and analog-digital converter chips (9) continuously filter and convert the analog measurement signal in a digital measurement signal,
- the microcontroller (10) continuously receive a digital measurement signal from the analog signal processing and analog-digital converter chips (9) and store said signal in a temporary memory (11), and
- the microprocessor (13) is periodically awakened from a sleep state to process the signal stored in the temporary memory.

15. Method according to claim 14, wherein the device is a device according to claim 12 or 13, and wherein if the microprocessor (13) detects that the signal recorded in the temporary memory is representative of a stimulation-ready state of the individual, the microprocessor (13) is continuously kept in an awaken state and command the acoustic transducer (23) and the codec module (24) to generate acoustic stimulations to the individual.
